# FASCICULE DE BREVET EUROPEEN

(11) **EP 3 305 092 B1**
(45) Date de publication et mention de la délivrance du brevet: **24.01.2024**
(21) Numéro de dépôt: 17203082.7
(22) Date de dépôt: 31.01.2014
(51) Int. Cl.: A23J 1/14

(54) **PROCEDE DE FRACTIONNEMENT DES SOLUBLES DE POIS**
FRAKTIONIERUNGSVERFAHREN VON LÖSLICHEN SUBSTANZEN VON ERBSEN
METHOD FOR FRACTIONING PEA SOLUBLES

(30) Priorité: 31.01.2013 FR 1350853
(43) Date de publication de la demande: 11.04.2018
(62) Demande divisionnaire de: 14706872.0
(73) Titulaire: Roquette Frères, 62136 Lestrem (FR)
(72) Inventeur: BARATA, Manuel, 62620 Maisnil les Ruitz (FR); DUFLOT, Pierrick, 62136 La Couture (FR); DHALLEINE, Claire, 59190 Caestre (FR); VERRIN, Jean-Marc, 62660 Beuvry (FR)
(74) Mandataire: Plasseraud IP

(56) Documents cités:
- WO-A1-2006/052003
- WO-A1-2012/116703
- FR-A1- 2 778 407
- FR-A1- 2 889 416
- US-A- 4 766 204
- LEI (LEIGH) GAO ET AL: "Pilot Scale Recovery of Proteins from a Pea Whey Discharge by Ultrafiltration", LWT - FOOD SCIENCE AND TECHNOLOGY, vol. 34, no. 3, 1 mai 2001 (2001-05-01), pages 149-158, XP055076942, ISSN: 0023-6438, DOI: 10.1006/fstl.2000.0743

## Description

La présente invention est relative à un procédé original de fractionnement des fractions solubles de pois, comprenant une étape de microfiltration par microfiltration tangentielle membranaire, suivie d'une étape d'ultrafiltration, et d'une éventuelle étape d'osmose inverse. On parvient ainsi à réduire la fuite de protéines vers les fractions solubles, à améliorer le rendement de l'étape unitaire de concentration par évaporation des fractions solubles, et on réussit à isoler de manière sélective les protéines d'intérêt. Il s'agit en outre d'un procédé simple à mettre en oeuvre, les dispositifs utilisés au niveau de chaque étape unitaire étant classiques et bien connus de l'homme du métier.

Depuis les années 70, le pois est la légumineuse à graines qui s'est le plus développée et Europe et majoritairement en France, notamment comme ressource protéique pour l'alimentation animale mais aussi humaine. Le pois contient environ 27 % en poids de matières protéiques. Le terme « pois » est ici considéré dans son acception la plus large et inclut en particulier toutes les variétés sauvages de « pois lisse » (« smooth pea »), et toutes les variétés mutantes de « pois lisse » et de « pois ridé » (« wrinkled pea »), et ce quelles que soient les utilisations auxquelles on destine généralement lesdites variétés (alimentation humaine, nutrition animale et/ou autres utilisations).

Parmi les constituants du pois, les plus valorisés actuellement sont l'amidon, les fibres et les protéines, encore désignés sous l'expression de constituants nobles. Le procédé de valorisation correspondant consiste à réaliser initialement un lait d'amidon, par mélange dans un pétrin entre la farine de pois et de l'eau. Après avoir extrait de ce lait l'amidon et les fibres, on dispose d'un produit riche en protéines. On effectue alors une opération de floculation sur le lait, qui dans le cadre de l'invention est effectuée par thermo coagulation, dont l'objectif est d'insolubiliser la ou les protéines d'intérêt. A ce stade du procédé, il est nécessaire de réaliser une séparation notamment par décantation centrifuge, de manière à isoler une composition très riche en protéines encore appelé « floc ». Le surnageant constitue ce que l'homme du métier désigne généralement sous l'expression de « fractions solubles ».

Il convient tout d'abord de préciser que le terme « fractions solubles » constitue un abus de langage en ce sens que ladite fraction contient un certain nombre de particules insolubles, comme des colloïdes divers et variés mais aussi et surtout des protéines. Ces fractions solubles doivent tout d'abord être concentrées par évaporation, de manière à ce qu'on puisse récupérer les insolubles (et notamment les protéines) qu'elles contiennent. A ce jour, les fractions solubles sont très peu exploitées ; elles sont utilisées de manière quasi exclusive comme source d'azote en fermentation et en tant qu'aliment nutritif pour le bétail une fois lesdites fractions enrichies en fibres.

La Demanderesse a récemment protégé, à travers les Demandes de Brevet français n° 09 51962 et n° 12 57680, un procédé d'extraction de β-amylases à partir d'une fraction soluble de plante amidonnière dont le pois, ledit procédé comprenant une étape de clarification par microfiltration et une étape de concentration / purification par ultrafiltration.

Or, force est de constater que le rendement du procédé global conduisant du pois initial à la composition riche en protéines (le floc) est loin d'atteindre les 100 %. On estime qu'à un échelon industriel, on retrouve au sein des fractions solubles entre 5 % et 25 %, mais plus généralement autour de 20 % en poids des protéines initialement contenues dans le pois de départ. Cette fuite massive de protéines, via les fractions solubles, n'est pas sans poser un certain nombre d'inconvénients.

Le premier d'entre eux est une perte sèche en protéines d'intérêt, qui sont actuellement uniquement valorisées à travers le floc de protéines issu de l'étape de décantation. Si on parvenait à isoler de manière globale la part de protéines contenue dans les fractions solubles, celle-ci pourrait avantageusement être incorporée ou redirigée dans le floc de protéines, en vue de l'enrichir.

Le deuxième problème posé par le procédé tel qu'il existe actuellement est un rendement très médiocre au niveau de l'étape unitaire de concentration par évaporation des fractions solubles : la richesse en protéines y est telle que le phénomène d'encrassement de l'évaporateur est très important et nécessite de fréquents arrêts pour nettoyage. En effet, de par leur sensibilité thermique, les protéines coagulent et ont une prédisposition à encrasser les dispositifs d'évaporation.

Enfin et selon un troisième éclairage, de récents travaux ont montré qu'une partie des protéines issue de la fraction soluble (la fraction dite PA1b) peut être avantageusement utilisée dans la fabrication d'un insecticide : ceci résulte de l'enseignement du document FR 2 778 407 A1. Il existe donc aussi un intérêt technique à isoler de manière sélective une ou des fractions protéiques, au sein-même des fractions solubles.

A la connaissance de la Demanderesse, cette problématique n'a jamais été abordée dans son ensemble, c'est-à-dire en vue de dégager des solutions visant à résoudre le triple problème technique tel qu'énoncé ci-dessus. Le document « Pilot scale recovery of proteins from a pea whey discharge by ultrafiltration » (Lei (Leigh) Gao, Khai D. Nguyen and Alphonsus C. Utioh, Lebensm.-Wiss. u.-Technol., vol 34, pp. 149-158, 2001) s'intéresse à la récupération de protéines de pois par centrifugation suivie d'ultrafiltration. La Demande de Brevet WO 2006 / 052003 concerne quant à elle un procédé de préparation de polypeptides solubles issus d'un effluent liquide de transformation de pois, par filtration membranaire puis séchage. La demande US 4 766 204 décrit un procédé de séparation des protéines et des carbohydrates présents dans des légumes, notamment les pois, dans lequel une fraction soluble de pois subit uniquement une étape d'ultrafiltration.

Travaillant à la recherche d'une solution globale pour optimiser le rendement du procédé d'extraction de protéines à partir du pois, la Demanderesse est parvenue à mettre au point un procédé qui résout le triple problème technique :
- de réduire la fuite de protéines vers les fractions solubles,
- d'améliorer le rendement de l'étape unitaire de concentration par évaporation des fractions solubles,
- d'isoler de manière sélective les protéines d'intérêt au sein des fractions solubles.

Ce procédé, qui est défini par les revendications, repose sur la succession de différentes étapes de séparation desdites fractions solubles, qui ont été obtenues comme décrit plus haut,
a) au moyen d'une microfiltration par microfiltration tangentielle membranaire,
b) puis d'une ultrafiltration, c) et enfin éventuellement d'une osmose inverse.

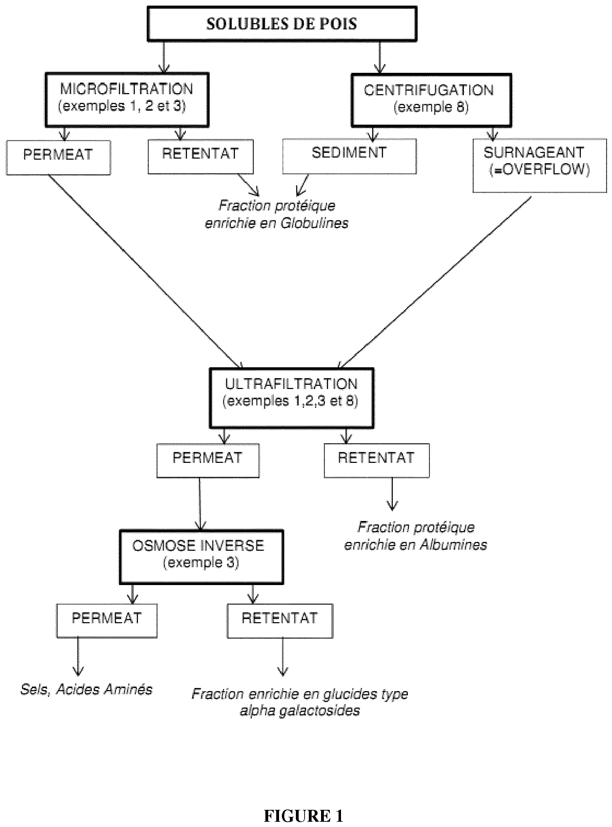

De manière tout à fait avantageuse, la mise en oeuvre de ces étapes de manière séquencée permet, au niveau de chaque unité de filtration, d'isoler une fraction protéique d'intérêt. Typiquement, les globulines et de l'acide phytique sont récupérés au niveau du rétentat de microfiltration ou du culot (ou sédiment) de centrifugation. Le perméat de microfiltration ou le surnageant de centrifugation subit ensuite une étape d'ultrafiltration : les albumines constituent alors l'essentiel de la matière sèche du rétentat d'ultrafiltration, le perméat étant destiné à subir l'éventuelle étape d'osmose inverse. Cette dernière permet d'isoler d'une part une fraction riche en glucides du type α-galactosides au niveau du rétentat, et d'autre part les acides aminés, glucides et autres sels présents dans le perméat. On pourra se référer au schéma de principe de la figure n° 1 de la présente Demande.

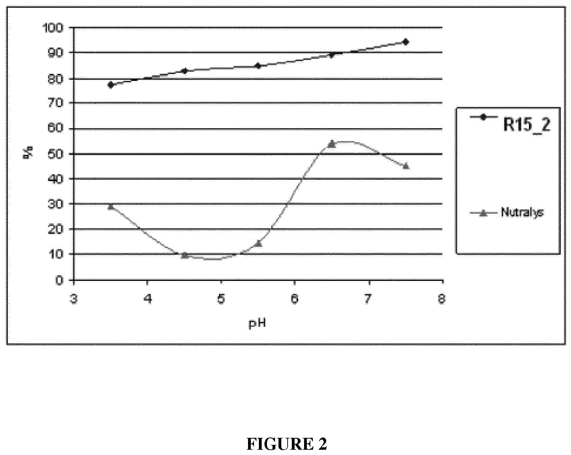

Les autres figures 2 à 5 illustrent les résultats et un profil électrophorétique obtenus dans les essais exemplifiés dans la présente demande.

On parvient donc à isoler de manière très précise différentes catégories de protéines d'intérêt, qui peuvent être valorisées par la suite dans une application donnée. De manière tout à fait avantageuse, le rétentat de microfiltration est riche en acide phytique. Or, cet acide inhibe l'absorption de divers cations (Zn, Cu, Co, Mn, Ca, Fe) en formant des sels insolubles (phytates). Cette propriété est exploitée en oenologie : le traitement au phytate de calcium est le seul qui soit autorisé (en France) pour déferrer les vins rouges
De manière avantageuse également, le rétentat issu de l'étape d'osmose inverse est particulièrement riche en α-galactoside, mais aussi en la fraction dite PA1b qui connaît une application directe dans la fabrication d'insecticides d'origine entièrement bio-sourcée (comme divulgué dans le document FR 2 778 407 précité). De plus, tout ou partie des fractions riches en les différentes protéines d'intérêt, et obtenues au niveau de chaque étape unitaire de filtration, peut être redirigée au niveau du floc, en aval de l'étape de décantation centrifuge. On augmente de ce fait la richesse du floc en protéines.

Enfin, le perméat final issu de l'étape d'osmose inverse qui contient avantageusement les acides aminés, glucides et autres sels, particules de très petites tailles, peut facilement être concentré au moyen d'un évaporateur, sans pour autant occasionner des problèmes de colmatage, qui nécessitent de fréquents arrêts de production pour des interventions de nettoyage, voire de changement des dispositifs.

Aussi, un premier objet de la présente invention consiste en un procédé de traitement des fractions solubles de pois obtenues au moyen de :
(i) la réalisation d'un lait d'amidon par mélange dans un pétrin entre la farine de pois et de l'eau.
(ii) la réalisation d'un produit riche en protéines par extraction de l'amidon et des fibres du lait d'amidon de l'étape (i) ;
(iii) la réalisation d'une floculation par thermo-coagulation sur le produit riche en protéines de l'étape (ii) ;
(iv) la séparation du produit issu de l'étape (iii) de manière à isoler un floc et un surnageant appelé fractions solubles,
ledit procédé comprenant :
a) une étape de microfiltration desdites fractions solubles par microfiltration tangentielle membranaire conduisant à un perméat de microfiltration,
b) suivie d'une étape d'ultrafiltration du perméat de microfiltration, conduisant à un perméat et à un rétentat d'ultrafiltration,
c) suivie d'une étape éventuelle d'osmose inverse du perméat d'ultrafiltration conduisant à un perméat et à un rétentat issus de l'osmose inverse.

Une des originalités de la présente invention consiste à faire subir aux fractions solubles de pois, compositions a priori non nobles, un certain nombre d'étapes de traitement en vue de valoriser leur contenu. Il convient de bien distinguer ce type de procédé où des étapes unitaires de traitement sont appliquées directement aux fractions solubles, des procédés de l'art antérieur qui peuvent recourir à ces mêmes étapes individuelles, mais en vue de traiter :
- soit les protéines de pois exemptes des fractions solubles en vue de purifier lesdites protéines de pois,
- soit ces mêmes protéines de pois encore chargées en fractions solubles et en vue justement de réaliser une séparation entre protéines et fractions solubles.

La première étape du procédé selon l'invention est donc une étape a) de microfiltration qui a lieu directement sur les fractions solubles de pois, telles qu'issues de l'étape de décantation centrifuge. Cette étape a notamment pour objectif d'isoler une fraction protéique riche en globulines.

La microfiltration des fractions solubles de pois conduit à un perméat et à un rétentat de microfiltration. C'est le perméat qui est ensuite traitée dans l'étape ultérieure b) d'ultrafiltration.

Cette étape de microfiltration est une microfiltration tangentielle membranaire. Plus particulièrement, la microfiltration tangentielle est préférentiellement réalisée avec des membranes céramiques présentant une porosité de 0,01 µm à 1 µm, préférentiellement de 0,05 µm à 0,5 µm.

De manière facultative, cette première étape de microfiltration peut être précédée d'une étape de floculation des particules insolubles contenues dans la fraction soluble de plantes amidonnières, par toute technique connue par ailleurs de l'homme du métier.

La deuxième étape du procédé selon l'invention consiste en une étape b) d'ultrafiltration, effectuée sur le perméat de microfiltration ou sur le surnageant de centrifugation. Elle permet d'obtenir d'une part un rétentat d'ultrafiltration riche en albumines, et d'autre part un perméat riche en la fraction dite PA1b dont il a déjà été mentionné qu'elle peut être valorisée dans la fabrication d'insecticide. Le perméat dans sa totalité peut donc être directement valorisé pour cette application insecticide ; il n'est pas nécessaire de le traiter/séparer outre mesure.

Plus particulièrement, il est recommandé de réaliser l'ultrafiltration à l'aide de membranes présentant un seuil de coupure compris entre 0.1 et 0.5 µm, la pression transmembranaire étant maintenue inférieure à 4 bars.

Enfin, le procédé selon la présente invention comprend une troisième étape c) qui reste éventuelle, mais qui est préférentiellement effectuée : il s'agit d'une osmose inverse réalisée sur le perméat d'ultrafiltration.

La Demanderesse recommande d'effectuer cette osmose avec des membranes présentant un seuil de coupure compris entre 100 Da et 500 Da.

Les exemples qui suivent permettent de mieux illustrer la Demande, sans toutefois en limiter la portée.

### EXEMPLES

### Exemple 1

Cet exemple illustre l'efficacité de combiner la microfiltration avec l'ultrafiltration pour obtenir dans un premier temps un rétentat de microfiltration riche en globulines, et dans un second temps un perméat d'ultrafiltration riche en glucides.

On commence tout d'abord par fabriquer la fraction soluble de pois.

De la farine de pois est initialement préparée par broyage de pois fourragers décortiqués sur broyeur à marteaux de type ALPINE équipé d'une grille de 100 pm. 300 kg de farine à 87% de matière sèche sont ensuite mis à tremper dans de l'eau à la concentration finale de 25% sur sec, à un pH de 6,5. 1044 kg de suspension de farine à 25% de matière sèche (soit donc 261 kg de farine sèche) sont alors introduits avec 500 kg d'eau dans une batterie d'hydrocyclones composée de 14 étages. Elle est alimentée par la suspension de farine à l'étage n° 5. Cette séparation conduit à l'obtention d'une phase légère qui correspond à la sortie de l'étage n° 1. Elle est constituée du mélange protéines, fibres internes et solubles.

Cette phase légère en sortie d'hydrocyclones renferme en mélange (142 kg sur sec au total) : les fibres (environ 14,8% en poids, soit 21 kg sec), les protéines (environ 42,8% en poids, soit 60,8 kg sec) et les solubles (environ 42,4% en poids, soit 60,2 kg sec). Cette fraction présente une matière sèche de 11,4 %. On procède à la séparation des fibres sur décanteurs centrifuges de type WESTFALIA employés dans une unité industrielle féculière de traitement de la pomme de terre. La phase légère en sortie de décanteur centrifuge renferme un mélange de protéines et de solubles, tandis que la phase lourde renferme les fibres de pois. La phase lourde renferme 105 kg de fibres à 20 % de matière sèche. On constate que la quasi- totalité des fibres est bien retrouvée dans cette fraction.

Quant à la fraction protéines et solubles, elle renferme 1142 kg d'un mélange en solution de solubles et de protéines (fraction à 6 % de matière sèche). On procède à la floculation des protéines à leur point isoélectrique par ajustement de la phase légère en sortie de décanteur centrifuge à un pH de 4,5 et chauffage à 50 °C.

Les protéines ainsi mises à floculer sont laissées 10 minutes en cuve de maturation. Après précipitation des protéines, on procède à une décantation centrifuge, qui permet de récupérer, après séchage, du sédiment renfermant 56 kg de protéines (86 % de Nx6,25 sur sec) à 93 % de matière sèche.

On procède ainsi jusqu'à obtenir 2500 litres de solubles de pois dont le pH est ajusté à 7.0 par ajout de soude à 50 %. La température de la suspension ainsi obtenue a été amenée à 70°C. La composition de la suspension ainsi obtenue (SOLF_1) est donnée dans le tableau 1.1.

**Tableau 1.1**

| | | SOLF_1 | |
|---|---|---|---|
| Teneur en matière sèche | | 2.5 | g pour 100 g |
| Teneur en protéine (Nx6.25) | | 27 | g pour 100 g de matière sèche |
| Teneur en cendres | | 16 | g pour 100 g de matière sèche |
| Teneur en glucides totaux | | 47 | g pour 100 g de matière sèche |
| Dont : | | | |
| | Raffinose | 4.0 | g pour 100 g de matière sèche |
| | Stachyose | 13.4 | g pour 100 g de matière sèche |
| | Verbacose | 15.1 | g pour 100 g de matière sèche |
| Autres | | 10 | g pour 100 g de matière sèche |

La solution est pompée au travers d'une unité de microfiltration équipée de membranes céramiques type Inside Ceram^{®} ayant un seuil de coupure de 0.14 µm (19 canaux de 4.5 mm). Tout au long de la filtration la température est régulée à 60°C et la pression transmembranaire maintenue à une valeur comprise entre 0.4 et 0.6 bar.

707 litres de perméat de microfiltration (P014_1) et 1768 litres de rétentat de microfiltration (R014_1) sont ainsi récupérés. Les compositions de chacune des fractions sont données dans le tableau 1.2.

**Tableau 1.2**

| | P014_1 | R014_1 | |
|---|---|---|---|
| Teneur en matière sèche | 2.5 | 2.5 | % |
| Teneur en protéine (Nx6.25) | 26.5 | 27.2 | g / 100 g de matière sèche |
| Teneur en cendres | 21.0 | 14.0 | g / 100 g de matière sèche |
| Teneur en glucides totaux | 45.0 | 47.0 | g / 100 g de matière sèche |
| Autres | 7.5 | 11.8 | g / 100 g de matière sèche |

550 litres du perméat (P014_1) sont pompés au travers d'une unité d'ultrafiltration. L'unité d'ultrafiltration est équipée de membranes céramiques type KERASEP^{®} BX commercialisées par la société NOVASEP et ayant un seuil de coupure de 15 Kda (7 canaux de 6 mm). Tout au long de la filtration la température est régulée à 60°C et la pression transmembranaire maintenue à une valeur comprise entre 1 et 3 bars.

467 litres de perméat d'ultrafiltration (P15_1) et 33 litres de rétentat (R15_1) sont ainsi récupérés. Les compositions de chacune des fractions sont données dans le tableau 1.3.

**Tableau 1.3**

| | P15_1 | R15_1 | |
|---|---|---|---|
| Teneur en matière sèche | 2.2 | 7.2 | % |
| Teneur en protéine (Nx6.25) | 15.0 | 75.1 | g / 100 g de matière sèche |
| Teneur en cendres | 23.6 | 10.3 | g / 100 g de matière sèche |
| Teneur en glucides totaux | 52.6 | 12.5 | g / 100 g de matière sèche |
| Autres | 8.8 | 2.1 | g / 100 g de matière sèche |

### Exemple 2

En comparaison avec l'exemple précédent, celui-ci illustre le bénéfice de la diafiltration pour optimiser la richesse du rétentat.

600 litres de solubles de pois issus de procédé de coagulation isoélectrique sont ajustés à un pH de 7.0 par ajout de soude à 50%. La température de la suspension ainsi obtenue a été amenée à 60°C. La composition de la suspension est celle donnée dans le tableau 1.1 précédent.

La solution est pompée au travers d'une unité de microfiltration équipée de membranes céramiques type Inside Ceram^{®} ayant un seuil de coupure de 0.14 µm (19 canaux de 4.5 mm). Tout au long de la filtration la température est régulée à 60°C et la pression transmembranaire maintenue à une valeur comprise entre 0.4 et 0.6 bar.

160 litres de perméat de microfiltration (P014_2) et 400 litres de rétentat de microfiltration (R014_2) sont ainsi récupérés.

110 litres du perméat (P014_2) sont pompés au travers d'une unité d'ultrafiltration. L'unité d'ultrafiltration est équipée de membranes céramiques type KERASEP^{®} BX commercialisées par la société NOVASEP et ayant un seuil de coupure de 15 Kda (7 canaux de 6mm). La fraction rétentat est maintenue en recyclage total sur l'alimentation et le perméat évacué. L'alimentation est additionnée par un débit permanent d'eau à raison d'un volume d'eau pour 7 volumes de perméat évacué. Tout au long de la filtration la température est régulée à 60°C et la pression transmembranaire maintenue à une valeur comprise entre 2 et 3 bars.

107 litres de perméat d'ultrafiltration (P15_2) et 7 litres de rétentat (R15_2) sont ainsi récupérés. Le rétentat (R15_2) est atomisé. La composition des fractions analysées est donnée dans le tableau 2.1.

**Tableau 2.1**

| | P15_2 | R15_2 | |
|---|---|---|---|
| Teneur en matière sèche | 3.3 | 11.0 | % |
| Teneur en protéine (Nx6.25) | 15.8 | 81.7 | g / 100 g de matière sèche |
| Teneur en cendres | 20.4 | 10.3 | g / 100 g de matière sèche |
| Teneur en glucides totaux | 51.0 | 6.8 | g / 100 g de matière sèche |
| Autres | 12.8 | 1.2 | g / 100 g de matière sèche |

### Exemple 3

Cet exemple illustre l'effet bénéfique de l'osmose inverse pour déminéraliser et augmenter la concentration en glucides totaux d'intérêt du perméat d'ultrafiltration.

150 litres de solubles de pois sont traités selon le protocole décrit dans l'exemple 2. 60 litres de perméat d'ultrafiltration (P15_3) sont ainsi obtenus puis pompés au travers d'une unité d'osmose inverse équipée de membranes organiques spiralées type OSMONICS DESAL^{®} commercialisées par la société General Electric Company et ayant un seuil de coupure de 350 Da. La fraction rétentat est maintenue en recyclage total sur l'alimentation et le perméat évacué. Tout au long de la filtration la température est régulée à 50°C et la pression maintenue à une valeur de l'ordre de 20 bars.

5 litres de rétentat (R350_3) sont ainsi récupérés. La composition des différentes fractions est donnée dans le tableau 3.1.

**Tableau 3.1**

| | | P15_3 | R350_3 | |
|---|---|---|---|---|
| Teneur en matière sèche | | 2.2 | 14 | g /100 g |
| Teneur en protéine (Nx6.25) | | 14.5 | 9 | g / 100 g de matière sèche |
| Teneur en cendres | | 20 | 6 | g / 100 g de matière sèche |
| Teneur en glucides totaux | | 56 | 65 | g / 100 g de matière sèche |
| | Raffinose | 4.5 | 6.0 | g / 100 g de matière sèche |
| | Stachyose | 18.0 | 19.1 | g / 100 g de matière sèche |
| | Verbacose | 16.9 | 21.0 | g / 100 g de matière sèche |
| Autres | | 16.5 | 12.0 | g / 100 g de matière sèche |

### Exemple 4

La solubilité en fonction du pH de la fraction atomisée R15_2 obtenue dans l'exemple 2 est mesurée. Elle est comparée à celle d'un isolat de protéine de pois type Nutralys^{®} F85M commercialisé par la Demanderesse.

La solubilité est considérée comme étant égale à la matière sèche d'un surnageant de centrifugation (15 min à 3000 g) d'une suspension à un pH donné et préparée par homogéinisation de 5 g de produit dans 100 g d'eau. La figure 2 représente la solubilité des 2 produits en fonction du pH. La fraction R15_2 a une meilleure solubilité que le Nutralys^{®} F85M. Cette meilleure solubilité est un avantage pour le secteur des formulations énergétiques dans le domaine du sport.

### Exemple 5

La capacité émulsifiante en fonction du pH de la fraction atomisée R15_2 obtenue dans l'exemple 2 est mesurée. Elle a été comparée à celle d'un isolat de protéine de pois type Nutralys^{®} F85M.

L'activité émulsifiante du produit est déterminée à partir du volume d'émulsion subsistant (en %), après dispersion dans un mélange eau-huile à un pH donné puis centrifugation. L'activité émulsifiante du produit est déterminée à partir du volume d'émulsion subsistant (en %), après dispersion dans un mélange eau-huile à un pH donné puis centrifugation selon la Méthode de YASUMATSU et al.(1972) décrite par : NACZK M., RUBIN L.J., SHAHIDI F., Functional properties and phytate content of pea protein préparations. Journal of Food Science, Volume 51 N°5, 1986, p 1245-1247.

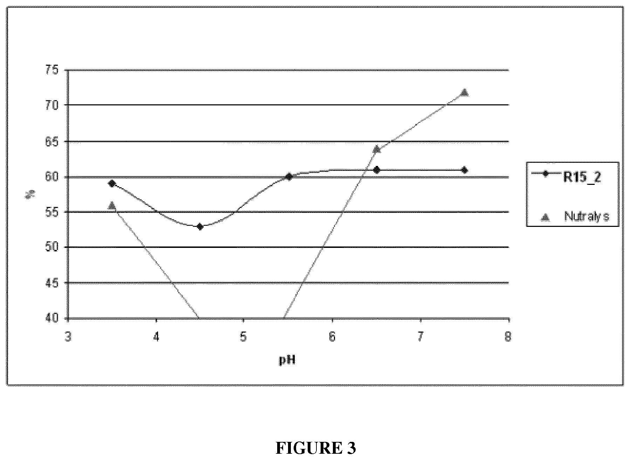

La figure 3 montre qu'à pH 4,5 et 5,5, la fraction R15_2 présente la meilleure capacité émulsifiante, pH où le Nutralys^{®} F85M n'a aucune capacité émulsifiante. Ces propriétés émlusifiantes sont valorisables entre autres dans le domaine de la charcuterie.

### Exemple 6

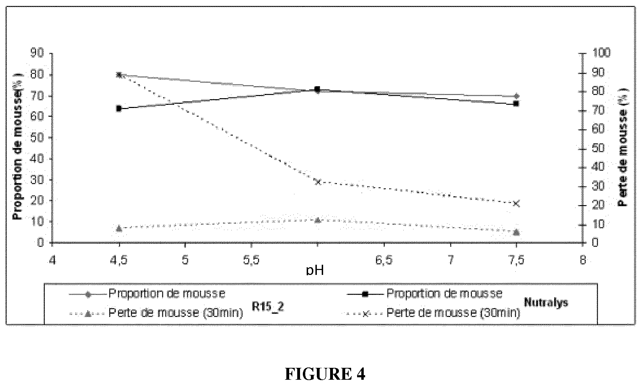

La capacité moussante et la stabilité de mousse de la fraction atomisée R15_2 obtenue dans l'exemple 2 est mesurée à différents pH. Elle est comparée à celle d'un isolat de protéine de pois type Nutralys^{®} F85M (voir figure 4).

La capacité moussante d'un échantillon de protéines est déterminée selon la méthode SUMNER A.K., NIELSEN M.A., YOUNGS C.G., Production and évaluation of pea protein isolate. Journal of Food Science, Volume 46, 1981, p 364-372. La perte de la mousse obtenue est calculée à partir du pourcentage de diminution du volume de mousse après 30 minutes de repos.

La tenue de la mousse de la fraction R15_2 est bien meilleure que celle du Nutralys^{®} F85M. Dans les crèmes glacées, les meringues, les cakes, le pain ou les barres hyperprotéinées, on cherchera effectivement une meilleure tenue en mousse.

### Exemple 7

Les qualités organoleptiques de la fraction atomisée R15_2 obtenue dans l'exemple 2 sont évaluées par un groupe de 20 panélistes. Elles sont comparées à celle d'un isolat de protéine de pois type Nutralys^{®} F85M.

Pour chaque essai 5 g de produit sont délayés dans 150 ml d'eau et maintenus au bainmarie à 50°C.

La fraction R15_2 est plus neutre en odeur et en goût en comparaison au Nutralys^{®} F85M.

Le tableau 7.1 énumère les descripteurs proposés par les panélistes lors de l'analyse.

**Tableau 7.1**

| | R15_2 | Nutralys^{®} F85M |
|---|---|---|
| Odeur | Neutre, quasi neutre | Végétal, fermenté |
| Goût | Neutre à presque neutre | Amer |

### Exemple 8

Le flux industriel d'une partie des solubles de pois préconcentrés à 10 % de matière sèche (SOPPr) est détourné des étapes de concentrations finales destinées à l'amener à 28 % de matière sèche pour alimenter un circuit pilote composé :
- d'une unité de décantation centrifuge type Westfalia^{®} CA-505 réglée sur une accélération de 3600 g et alimentée à environ 12 m³/h. Celle-ci génère en continu 11 m³/h d'une fraction overflow (OVF), réintroduits dans le circuit de concentration des solubles en aval du point de prélèvement de la fraction SOPPr, et 600 kg/h de sédiment (SED) mélangé à l'isolat de protéine avant séchage.
- d'une unité d'ultrafiltration équipée de membranes céramiques type KERASEP^{®} BW commercialisées par la société NOVASEP et ayant un seuil de coupure de 5 KDa (19 canaux de 3.5 mm). Celle-ci est alimentée de manière discontinue par une fraction du volume d'overflow (OVF) généré par l'étape précédente.

La fraction rétentat est maintenue en recyclage total sur l'alimentation et le perméat évacué. L'alimentation est additionnée par un débit permanent d'eau à raison d'un volume d'eau pour 5 volumes de perméat évacué. Tout au long de la filtration la température est régulée à 60°C et la pression transmembranaire maintenue à une valeur comprise entre 2 et 3 bars. 2 fractions sont générées : rétentat (RET) et perméat (PERM).

La composition des différentes fractions est donnée dans les tableaux 8.1 et 8.2.

**Tableau 8.1**

| | | SOPPr | OVF | SED | |
|---|---|---|---|---|---|
| Teneur en matière sèche | | 10 | 8 | 25 | g / 100 g |
| Teneur en protéine (Nx6.25) | | 30 | 29 | 72 | g / 100 g de matière sèche |
| Teneur en cendres | | 13 | 14 | 4 | g / 100 g de matière sèche |
| Teneur en glucides totaux | | 44 | 45 | 12 | g / 100 g de matière sèche |
| | Raffinose | 5 | 4 | - | g / 100 g de matière sèche |
| | Stachyose | 14 | 13 | - | g / 100 g de matière sèche |
| | Verbacose | 13 | 15 | - | g / 100 g de matière sèche |
| Autres | | 13 | 12 | 12 | g / 100 g de matière sèche |

**Tableau 8.2**

| | | RET | PERM | |
|---|---|---|---|---|
| Teneur en matière sèche | | 14 | 5 | g / 100 g |
| Teneur en protéine (Nx6.25) | | 84 | 3 | g / 100 g de matière sèche |
| Teneur en cendres | | 5 | 19 | g / 100 g de matière sèche |
| Teneur en glucides totaux | | 3 | 61 | g / 100 g de matière sèche |
| | Raffinose | - | 5 | g / 100 g de matière sèche |
| | Stachyose | - | 18 | g / 100 g de matière sèche |
| | Verbacose | - | 20 | g / 100 g de matière sèche |
| Autres | | 8 | 17 | g / 100 g de matière sèche |

### Exemple 9

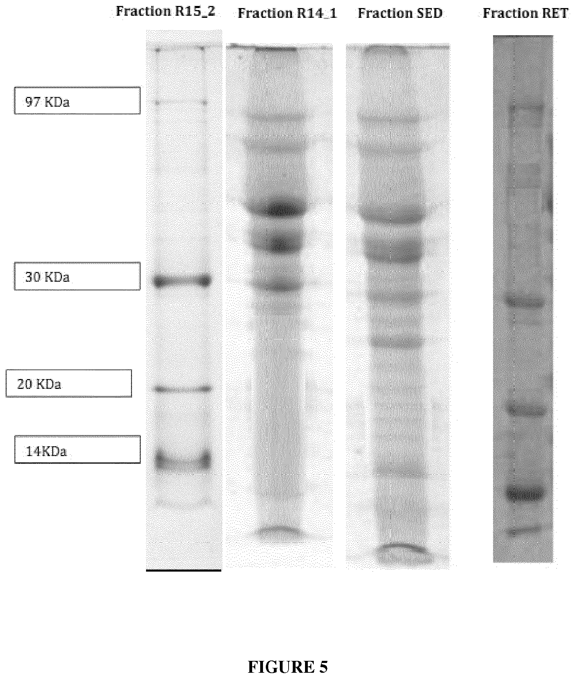

Un profil électrophorétique, présenté sur la figure 5, est déterminé sur la fraction atomisée R15_2 obtenue dans l'exemple 2, la fraction R014_1 obtenue dans l'exemple 1, les fractions SED et RET obtenues dans l'exemple 8. Le profil est effectué par migration de l'échantillon en conditions dénaturantes et réductrices au travers d'un gel de polyacrylamide à 20 % suivie d'une coloration au bleu de Coomassie. Les profils correspondants apparaissent en figure 5.

Pour les fractions R15_2 et RET, on note la présence marquée de bandes aux alentours de 14 kDa correspondant aux albumines PA1, la bande à 30 kDa correspondant aux dimères d'albumines PA2. Ceci en fait une fraction particulièrement intéressante pour être valorisée selon les indications du document FR 2 778 407 A1.

Pour les fractions R014_1 et RET, on constate une prédominance de bandes aux alentours des 50 kDa et au-delà, reflétant la présence importante de globulines, ce qui lui permet d'être valorisé entre autres en nutrition animale.

### Exemple 10

Cet exemple illustre l'effet bénéfique du prétraitement des solubles sur la durée des cycles et donc de la disponibilité de l'étape de concentration.

Le fonctionnement de l'unité de décantation centrifuge décrit dans l'exemple 8 est maintenu constant de manière à observer l'impact sur la durée de cycle de fonctionnement de l'étape de concentration des solubles. Les données sont ainsi comparées à celles de cette étape de concentration sans prétraitement des solubles par l'unité de décantation. Dans les 2 cas de figure, les paramètres de conduite du système de concentration (débit vapeur, vide, température produit, matière sèches d'entrée et de sortie des solubles) sont maintenus constants. La durée de cycle est définie par l'intervalle de temps s'écoulant entre 2 arrêts du système d'évaporation pour lavage. Le lavage a été initié dès que le débit de condensats d'évaporation diminue de plus de 15 % par rapport à sa valeur nominale obtenue après nettoyage complet (> 40 m³/h pour un débit alimentation en solubles de 55 m3/h).

**Tableau 10.1**

| | | Durée cycles (h) |
|---|---|---|
| Sans prétraitement des solubles concentrés | Cycle 1 | 72 |
| | Cycle 2 | 65 |
| | Cycle 3 | 68 |
| Avec prétraitement des solubles concentrés | Cycle 1 | 86 |
| | Cycle 2 | 90 |
| | Cycle 3 | 89 |

## Revendications

1. Procédé de traitement des fractions solubles de pois comprenant :
a) une étape de microfiltration desdites fractions solubles conduisant à un perméat de microfiltration,
b) suivie d'une étape d'ultrafiltration du perméat de microfiltration, conduisant à un perméat et à un rétentat d'ultrafiltration,
c) suivie d'une étape éventuelle d'osmose inverse du perméat d'ultrafiltration conduisant à un perméat et à un rétentat issus de l'osmose inverse,
**caractérisé en ce que** l'étape a) de microfiltration est une microfiltration tangentielle membranaire, et
ledit procédé comprenant avant l'étape a) une étape de préparation des fractions solubles comprenant :
(i) la réalisation d'un lait d'amidon par mélange dans un pétrin entre la farine de pois et de l'eau,
(ii) la réalisation d'un produit riche en protéines par extraction de l'amidon et des fibres du lait d'amidon de l'étape (i),
(iii) la réalisation d'une floculation par thermo-coagulation sur le produit riche en protéines de l'étape (ii) ;
(iv) la séparation du produit issu de l'étape (iii) de manière à isoler un floc et un surnageant appelé fractions solubles.

2. Procédé selon la revendication 1, **caractérisé en ce que** la microfiltration tangentielle membranaire est réalisée avec des membranes céramiques présentant une porosité de 0,01 µm à 1 µm, préférentiellement de 0,05 µm à 0,5 µm.

3. Procédé selon l'une quelconque des revendications 1 à 2, **caractérisé en ce que** l'étape a) est précédée par une étape de floculation des particules insolubles contenues dans la fraction soluble de pois.

4. Procédé selon l'une quelconque des revendications 1 à 3, **caractérisé en ce que** l'étape b) d'ultrafiltration est réalisée à l'aide de membranes présentant un seuil de coupure compris entre 0.1 et 0.5 µm, la pression transmembranaire étant maintenue inférieure à 4 bars.

5. - Procédé selon l'une quelconque des revendications 1 à 4, **caractérisé en ce que** l'étape c) d'osmose inverse est mise en oeuvre.

6. - Procédé selon la revendication 5, **caractérisé en ce que** l'osmose inverse est réalisée avec des membranes présentant un seuil de coupure compris entre 100 Da et 500 Da.

## Patentansprüche

1. Verfahren zur Verarbeitung löslicher Fraktionen von Erbsen, umfassend:
a) einen Schritt der Mikrofiltration der genannten löslichen Fraktionen, der zu einem Mikrofiltrationspermeat führt,
b) gefolgt von einem Schritt der Ultrafiltration des Mikrofiltrationspermeats, der zu einem Ultrafiltrationspermeat und einem Ultrafiltrationsretentat führt,
c) gefolgt von einem möglichen Schritt der Umkehrosmose des Ultrafiltrationspermeats, der zu einem Permeat und einem Retentat aus der Umkehrosmose führt,
**dadurch gekennzeichnet, dass** der Schritt der Mikrofiltration a) eine tangentiale Membranmikrofiltration ist, und
besagtes Verfahren umfaßt vor dem Schritt a) einen Schritt zur Herstellung von löslichen Fraktionen, umfassend
(i) Herstellen einer Stärkemilch durch Mischen von Erbsenmehl und Wasser in einem Kneter,
(ii) Herstellen eines proteinreichen Produkts durch Extraktion der Stärke und der Fasern aus der Stärkemilch aus Schritt (i),
(iii) Durchführung von Ausflocken durch Thermokoagulation des proteinreichen Produkts aus Schritt (ii),
(iv) Abtrennen des Produkts aus Schritt (iii), wobei Flocken und ein Überstand, der als lösliche Fraktionen bezeichnet wird, isoliert werden.

2. Verfahren nach Anspruch 1, **dadurch gekennzeichnet, dass** die tangentiale Membranmikrofiltration mit keramischen Membranen durchgeführt wird, die eine Porosität von 0,01 µm bis 1 µm, vorzugsweise von 0,05 µm bis 0,5 µm aufweisen.

3. Verfahren nach einem der Ansprüche 1 oder 2, **dadurch gekennzeichnet, dass** Schritt a) ein Schritt der Ausflockung der in der löslichen Erbsenfraktion enthaltenen unlöslichen Partikel vorausgeht.

4. Verfahren nach einem der Ansprüche 1 bis 3, **dadurch gekennzeichnet, dass** der Schritt der Ultrafiltration b) mit Hilfe von Membranen mit einer Ausschlussgrenze zwischen 0,1 und 0,5 µm durchgeführt wird, wobei der Transmembrandruck auf unter 4 bar gehalten wird.

5. Verfahren nach einem der Ansprüche 1 bis 4, **dadurch gekennzeichnet, dass** Schritt c) der Umkehrosmose durchgeführt wird.

6. Verfahren nach Anspruch 5, **dadurch gekennzeichnet, dass** die Umkehrosmose mit Membranen durchgeführt wird, die eine Ausschlussgrenze zwischen 100 Da und 500 Da aufweisen.

## Claims

1. - A method for treating pea soluble fractions, comprising:
a) a step of microfiltration of said soluble fractions resulting in a microfiltration permeate,
b) followed by a step of ultrafiltration of the microfiltration permeate, resulting in an ultrafiltration permeate and an ultrafiltration retentate,
c) followed by an optional step of reverse osmosis of said ultrafiltration permeate, resulting in a permeate and a retentate from the reverse osmosis,
**characterized in that** the microfiltration step a) is a tangential membrane microfiltration,
said method comprising before the step a) a step of preparation of soluble fractions comprising
(i) the preparation of a starch milk, by mixing pea flour and water in a kneading machine,
(ii) the preparation of a protein-rich product by extraction of the starch and the fibers from said starch milk from step i),
(iii) a flocculation operation by thermocoagulation on said protein-rich product from step ii);
(iv) a separation operation of the product obtained from step iii) to obtain a floc and a supernatant called soluble fractions.

2. - The method as claimed in claim 1, **characterized in that** the tangential membrane microfiltration is carried out with ceramic membranes having a porosity of 0.01 µm to 1 µm, preferentially of 0.05 µm to 0.5 µm.

3. - The method as claimed in any one of claims 1 to 2, **characterized in that** step a) is preceded by a step of flocculation of the insoluble particles contained in the pea soluble fraction.

4. - The method as claimed in any one of claims 1 to 3, **characterized in that** the ultrafiltration step b) is carried out using membranes which have a cut-off threshold of between 0.1 and 0.5 µm, the transmembrane pressure being maintained below 4 bar.

5. - The method as claimed in any one of claims 1 to 4, **characterized in that** the reverse osmosis step c) is carried out.

6. - The method as claimed in claim 5, **characterized in that** the reverse osmosis is carried out with membranes having a cut-off threshold of between 100 Da and 500 Da.
